(19)

**Europäisches Patentamt**
**European Patent Office**
**Office européen des brevets**

(11)    **EP 1 753 804 B1**

(12)    **EUROPÄISCHE PATENTSCHRIFT**

(45) Veröffentlichungstag und Bekanntmachung des Hinweises auf die Patenterteilung:
**30.04.2008 Patentblatt 2008/18**

(51) Int Cl.:
*C08G 77/04* (2006.01)    *D21H 19/32* (2006.01)
*D06M 15/643* (2006.01)

(21) Anmeldenummer: **05748686.2**

(22) Anmeldetag: **02.06.2005**

(86) Internationale Anmeldenummer:
**PCT/EP2005/005952**

(87) Internationale Veröffentlichungsnummer:
**WO 2005/121218 (22.12.2005 Gazette 2005/51)**

(54) **VERFAHREN ZUR MODIFIZIERUNG FASERARTIGER SUBSTRATE MIT SILOXANCOPOLYMEREN**

METHOD FOR MODIFYING FIBROUS SUBSTRATES WITH SILOXANE COPOLYMERS

PROCEDE POUR MODIFIER DES SUBSTRATS FIBREUX AVEC DES COPOLYMERES DE SILOXANES

(84) Benannte Vertragsstaaten:
**DE**

(30) Priorität: **11.06.2004 DE 102004028322**

(43) Veröffentlichungstag der Anmeldung:
**21.02.2007 Patentblatt 2007/08**

(73) Patentinhaber: **Wacker Chemie AG**
**81737 München (DE)**

(72) Erfinder:
• **HERZIG, Christian**
**83329 Waging (DE)**
• **BECKER, Richard**
**84489 Burghausen (DE)**

• **BURGER, Willibald**
**84489 Burghausen (DE)**
• **HOHBERG, Thomas**
**9472 Grabs (DE)**

(74) Vertreter: **Deffner-Lehner, Maria et al**
**Wacker Chemie AG**
**Zentralbereich Patente, Marken und Lizenzen**
**Hanns-Seidel-Platz 4**
**81737 München (DE)**

(56) Entgegenhaltungen:
**EP-A- 0 473 812        US-A1- 2003 032 751**
**US-B1- 6 524 564**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Modifizierung faserartiger Substrate mit Siloxancopolymeren, wie die Modifizierung natürlicher oder künstlicher Substrate mit Faserstruktur.

US-A 5,001,210 beschreibt eine Methode zur Herstellung von Polyurethanen, bei der aminofunktionelle Siloxantelechele nach Umsatz mit cyclischen Carbonaten mit Di- oder Poly-isocyanaten in die Zielprodukte überführt werden. Polyether werden in Form von Diaminopolyethern verwendet, die im Vergleich zu Polyetherdiolen und -monoolen teuer sind.

In EP-A 1 178 069 ist die Herstellung von Polyetherurethanzwischenstufen beschrieben, indem Alkenylpolyether mit Diisocyanaten umgesetzt werden und an diese Silane, die hydrolyseempfindliche Gruppen tragen, addiert werden. Siloxankettenpolymere sind so nicht erhältlich.

Verzweigte Polyethersiloxane sind aus Chemical Abstracts 136: 38808 bekannt. Hydrogensiloxane werden mit Divinylsiloxanen und Allylpolyether gleichzeitig umgesetzt. Überschüssige Polyethermengen verbleiben ungebunden im Produktgemisch. Die Produkte werden als Textilweichmacher verwendet und sind frei von Urethan- und Harnstoffgruppen.

In US 2003/0032726 bzw. der korrespondierenden WO 02/088209 (A. Andrew Shores) ist ein Reaktionsprodukt beschrieben aus (A) Polyisocyanat, (B) Silicon, das ein Dimethylpolysiloxansegment und eine oder mehrere Isocyanat-reaktive Gruppen enthält, (C) Reaktant mit einer oder mehreren Isocyanat-reaktiven Gruppen und einer oder mehreren ionisierbaren Gruppen und (D) gegebenenfalls einer organischen Substanz, die eine oder mehrere Isocyanat-reaktive Gruppen aber keine ionisierbare Gruppen enthält und (E) Verbindung, die das Gegenion für genannte ionisierbare Gruppen liefert, wobei entweder das Silicon (B) oder der Reaktant (C) oder beide eine einzige Isocyanat-reaktive Gruppe enthält. Das Reaktionsprodukt ist geeignet als Formentrennmittel, als Schutzfilm, Hydrophobiermittel für Beton und Mauerwerk oder wasserabweisende Beschichtung auf Papier und Textilien.

In US 2003/0032751 (A. Andrew Shores) ist ein Reaktionsprodukt beschrieben aus (A) Polyisocyanat, (B) Silicon, das ein Dimethylpolysiloxansegment und eine oder mehrere Isocyanat-reaktive Gruppen enthält, (C) Reaktant mit einer oder mehreren Isocyanat-reaktiven Gruppen und einer oder mehreren ionisierbaren Gruppen und (D) gegebenenfalls einer organischen Substanz, die eine oder mehrere Isocyanat-reaktive Gruppen aber keine ionisierbare Gruppen enthält und (E) Verbindung, die das Gegenion für genannte ionisierbare Gruppen liefert, wobei das durchschnittliche Molekulargewicht des Reaktionsproduktes 600 bis 20 000 ist. Als Anwendungen werden die gleichen genannt wie in US 2003/0032726.

Es bestand die Aufgabe Siloxancopolymere bereitzustellen, die geeignet sind, faserartige Substrate, wie natürliche oder künstliche Substrate mit Faserstruktur, insbesondere textile Flächengebilde, weich und gleichzeitig hydrophil auszurüsten. Weiterhin bestand die Aufgabe, dass diese Siloxancopolymere in einem einfachen Verfahren herstellbar sind und in Wasser leicht zu dispergieren sind, insbesondere selbstdispergierend sind, d.h. ohne Mitverwendung von Emulgatoren eine Emulsion, insbesondere Microemulsion, bilden. Die Aufgabe wird durch die Erfindung gelöst.

Gegenstand der Erfindung ist ein Verfahren zur Modifizierung faserartiger Substrate mit Siloxancopolymeren herstellbar indem in einem ersten Schritt Organopolysiloxane (1), die pro Molekül mindestens ein Si-gebundenes Wasserstoffatom, vorzugsweise mindestens zwei Si-gebundene Wasserstoffatome, aufweisen, mit weitgehend linearen oligomeren oder polymeren Verbindungen (2) der allgemeinen Formel

$$R^1\text{-}(A\text{-}C_nH_{2n})_m\text{-}A^1\text{-}H \qquad (I),$$

wobei $R^1$ einen einwertigen gegebenenfalls substituierten Kohlenwasserstoffrest bedeutet, an den Si-H-Gruppen in einer Hydrosilylierungsreaktion angelagert werden können, vorzugsweise einen aliphatische C-C-Mehrfachbindung aufweisenden Kohlenwasserstoffrest bedeutet,

A einen zweiwertigen, polaren organischen Rest ausgewählt aus der Gruppe von -O- , -C(O)-O- , -O-C(O)- , -O-C(O)-O- , -C(O)-NH- , -NH-C(O)- , Urethanrest und Harnstoffrest, vorzugsweise ein Sauerstoffatom -O-, bedeutet,

$A^1$ einen zweiwertigen, polaren organischen Rest ausgewählt aus der Gruppe von -O- , -NH- und -NR'- (wobei R' einen einwertigen Kohlenwasserstoffrest mit 1 bis 18 Kohlenstoffatomen bedeutet), vorzugsweise ein Sauerstoffatom -O-, bedeutet,

n eine ganze Zahl von 1 bis 20, vorzugsweise 1 bis 4, bevorzugt 2 oder 3, ist und

m gleich 0 oder eine ganze positive Zahl, vorzugsweise 5 bis 50, ist,

umgesetzt werden,

und in einem zweiten Schritt

die so erhaltenen H-$A^1$-Gruppen aufweisenden Zwischenprodukte (3) mit organischen Verbindungen (4), die pro Molekül mindestens zwei Isocyanatgruppen aufweisen, umgesetzt werden.

Vorzugsweise ist der Wassergehalt der zur Herstellung der erfindungsgemäßen Siloxancopolymere eingesetzten Verbindungen (1) und (2) niedriger ist als 2000 Gew.-ppm, vorzugsweise niedriger ist als 1500 Gew.-ppm, bevorzugt niedriger ist als 1000 Gew.-ppm, jeweils bezogen auf das Gesamtgewicht von Verbindungen (1) und (2).

Der Wassergehalt bezieht sich auf Raumtemperatur (20°C) und den Druck der umgebenden Atmosphäre (1020 hPa).

Der Begriff faserartige Substrate soll im Rahmen der vorliegenden Erfindung alle natürlichen oder künstlichen Substrate mit Faserstruktur umfassen.

Der Begriff Modifizierung faserartiger Substrate soll im Rahmen der vorliegenden Erfindung die Behandlung oder Imprägnierung faserartiger Substrate, um ihre Eigenschaften in gewünschter Weise zu verändern, umfassen, beispielsweise sollen die faserartigen Substrate weich und hydrophil ausgerüstet werden.

Die erfindungsgemäßen Siloxancopolymere besitzen eine Viskosität von vorzugsweise 1 000 bis 100 000 000 mPa·s bei 25°C, bevorzugt 10 000 bis 10 000 000 mPa·s bei 25°C.

Im ersten Verfahrensschritt werden als Organopolysiloxane (1) vorzugsweise lineare, cyclische oder verzweigte Organopolysiloxane aus Einheiten der allgemeinen Formel

$$R_e H_f SiO_{\frac{4-e-f}{2}} \qquad (II),$$

wobei

R gleich oder verschieden sein kann und einen einwertigen, gegebenenfalls substituierten Kohlenwasserstoffrest mit 1 bis 18 Kohlenstoffatom(en) je Rest bedeutet,
e 0, 1, 2 oder 3,
f 0, 1 oder 2

und die Summe von e+f 0, 1, 2 oder 3 ist,
mit der Maßgabe, dass pro Molekül mindestens ein Si-gebundenes Wasserstoffatom, bevorzugt mindestens 2 Si-gebundene Wasserstoffatome vorliegen, verwendet.

Bevorzugt werden als Organopolysiloxane (1) solche der allgemeinen Formel

$$H_g R_{3-g} SiO(SiR_2O)_o(SiRHO)_p SiR_{3-g}H_g \qquad (III)$$

wobei R die oben dafür angegebene Bedeutung hat,

g 0, 1 oder 2,
o 0 oder eine ganze Zahl von 1 bis 1500 und
p 0 oder eine ganze Zahl von 1 bis 200 ist,

mit der Maßgabe, dass pro Molekül mindestens ein Si-gebundenes Wasserstoffatom, bevorzugt mindestens zwei Si-gebundene Wasserstoffatome, vorliegen, verwendet.

Im Rahmen dieser Erfindung soll Formel (III) so verstanden werden, dass o Einheiten -(SiR$_2$O)- und p Einheiten -(SiRHO)- in beliebiger Weise im Organopolysiloxanmolekül verteilt sein können.

Besonders bevorzugt ist g in Formel (III) 1 und p in Formel (III) 0 und es werden als Organopolysiloxane (1) $\alpha,\omega$-Dihydrogenpolydiorganosiloxane, insbesondere $\alpha,\omega$-Dihydrogenpolydimethylsiloxane, eingesetzt.

Die Organopolysiloxane (1) besitzen vorzugsweise eine durchschnittliche Viskosität von 10 bis 1 000 mPa.s bei 25°C, bevorzugt 50 bis 1000 mPa.s bei 25°C und besonders bevorzugt 60 bis 600 mPa.s bei 25°C.

Beispiele für Reste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der $\alpha$- und der $\beta$-Phenylethylrest.

Beispiele für substituierte Reste R sind Halogenalkylreste, wie der 3,3,3-Trifluor-n-propylrest, der 2,2,2,2',2', 2'-Hexafluorisopropylrest, der Heptafluorisopropylrest und Halogenarylreste, wie der o-, m- und p-Chlorphenylrest.

Bevorzugt handelt es sich bei dem Rest R um einen einwertigen Kohlenwasserstoffrest mit 1 bis 6 Kohlenstoffatomen, wobei der Methylrest besonders bevorzugt ist.

Beispiele für Reste R gelten im vollen Umfang für Reste R'.

$R^1$ bedeutet vorzugsweise einen einwertigen, Kohlenwasserstoffrest mit aliphatischer C-C-Mehrfachbindung.

Beispiele für Reste $R^1$ sind Alkenylreste, wie der Vinyl-, 5-Hexenyl-, Cyclohexenyl-, 1-Propenyl-, Allyl-, 3-Butenyl- und 4-Pentenylrest, und Alkinylreste, wie der Ethinyl-, Propargyl- und 1-Propinylrest.

Bevorzugt handelt es sich bei dem Rest $R^1$ um einen Alkenylreste, insbesondere ω-Alkenylrest, wobei der Allylrest besonders bevorzugt ist.

Bevorzugt als oligomere oder polymere Verbindungen (2) sind aliphatisch ungesättigte Alkohole der allgemeinen Formel

$$H_2C=CH-R^2-(OC_nH_{2n})_m-OH \qquad (IV),$$

wobei $R^2$ ein zweiwertiger Kohlenwasserstoffrest mit 1 bis 20 Kohlenstoffatomen, bevorzugt ein Rest der Formel $-CH_2-$, $-CH(CH_3)-$ oder $-C(CH_3)_2-$ ist und

n und m die oben dafür angegebene Bedeutung haben.

Bevorzugte Beispiele für Polyether (2) sind solche der allgemeinen Formel

$$H_2C=CH-R^2-(OCH_2CH_2)_a[OCH_2CH(CH_3)]_b-OH \qquad (IV'),$$

wobei $R^2$ die oben dafür angegebene Bedeutung hat und

a und b 0 oder eine ganze Zahl von 1 bis 200 ist, mit der Maßgabe, dass die Summe a+b mindestens 1 ist, vorzugsweise 5 bis 50 ist.

Weitere Beispiele für oligomere oder polymere Verbindungen (2) sind ungesättigte Polyester, wie $H_2C=CH-R^2-[O(O)CC_nH_{2n}]_m-OH$, ungesättigte Polycarbonate, wie $H_2C=CH-R^2-(OC(O)OC_nH_{2n}]_m-OH$ und ungesättigte Polyamide, wie $H_2C=CH-R^2-[NHC(O)C_nH_{2n}]_m-NH_2$,

wobei $R^2$, n und m die oben dafür angegebene Bedeutung haben. Bevorzugt als monomere Verbindung (2) sind ungesättigte Verbindungen der Formel

$$H_2C=CH-R^2-OH,$$

wobei $R^2$ in der obigen Bedeutung ist und in diesem Fall bevorzugt ein Rest der Formel

$$-(CH_2)_n-$$

mit n in beschriebener Bedeutung. Bevorzugte monomere Verbindungen (2) sind Allylalkohol, 5-Hexenol und 7-Octenol.

Die Verbindungen (2) werden im ersten Verfahrensschritt vorzugsweise in Mengen von 1,0 bis 4,0, bevorzugt 1,3 bis 2,5 Mol Rest $R^1$, der vorzugsweise ein Rest mit aliphatischer C-C-Mehrfachbindung ist, bevorzugt ein ω-Alkenylrest ist, je Grammatom Si-gebundenem Wasserstoff im Organopolysiloxan (1) eingesetzt. Überschüssig eingesetzte monomere Verbindung (2) kann entweder im Reaktionsgemisch belassen werden oder destillativ teilweise oder ganz entfernt werden, soweit es deren Flüchtigkeit erlaubt.

Im ersten Verfahrensschritt werden vorzugsweise die Anlagerung von Si-gebundenem Wasserstoff an aliphatische Mehrfachbindung fördernde Katalysatoren (5) eingesetzt. Als Katalysatoren (5) können auch bei dem erfindungsgemäßen Verfahren die gleichen Katalysatoren eingesetzt werden, die auch bisher zur Förderung der Anlagerung von Si-gebundenem Wasserstoff an aliphatische Mehrfachbindung eingesetzt werden konnten. Bei den Katalysatoren handelt es sich vorzugsweise um ein Metall aus der Gruppe der Platinmetalle oder um eine Verbindung oder einen Komplex aus der Gruppe der Platinmetalle. Beispiele für solche Katalysatoren sind metallisches und feinverteiltes Platin, das sich auf Trägern, wie Siliciumdioxid, Aluminiumoxid oder Aktivkohle befinden kann, Verbindungen oder Komplexe von Platin, wie Platinhalogenide, z.B. $PtCl_4$, $H_2PtCl_6 \ast 6H_2O$, $Na_2PtCl_4 \ast 4H_2O$, Platin-Olefin-Komplexe, Platin-Alkohol-Komplexe, Platin-Alkoholat-Komplexe, Platin-Ether-Komplexe, Platin-Aldehyd-Komplexe, Platin-Keton-Komplexe, einschließlich Umsetzungs-produkten aus $H_2PtCl_6 \ast 6H_2O$ und Cyclohexanon, Platin-Vinyl-siloxankomplexe, wie Platin-1,3-Divinyl-1,1,3,3-tetramethyl-disiloxankomplexe mit oder ohne Gehalt an nachweisbarem anorganisch gebundenem Halogen, Bis-(gamma-picolin)-platindichlorid, Trimethylendipyridinplatindichlorid, Dicyclopentadienplatindichlorid, Dimethylsulfoxydethylenplatin-(II)-di-chlorid, Cyclooctadien-Platindichlorid, Norbornadien-Platindichlorid, Gamma-picolin-Platindichlorid, Cyclopentadien-Platindichlorid, sowie Umsetzungsprodukte von Platintetrachlorid mit Olefin und primärem Amin oder sekundärem Amin oder primärem und sekundärem Amin, wie das Umsetzungsprodukt aus in 1-Octen gelöstem Platintetrachlorid mit sec.-Butylamin oder Ammonium-Platinkomplexe.

Der Katalysator (5) wird im ersten Verfahrensschritt vorzugsweise in Mengen von 1 bis 50 Gew.-ppm (Gewichtsteilen je Million Gewichtsteilen), bevorzugt in Mengen von 2 bis 20 Gew.-ppm, jeweils berechnet als elementares Platin und bezogen auf das Gesamtgewicht der Organopolysiloxane (1) und Verbindungen (2) eingesetzt.

Der erste Verfahrensschritt wird vorzugsweise beim Druck der umgebenden Atmosphäre, also etwa bei 1020 hPa (abs.), durchgeführt, es kann aber auch bei höheren oder niedrigeren Drücken durchgeführt werden. Ferner wird der erste Verfahrensschritt vorzugsweise bei einer Temperatur von 60°C bis 140°C, bevorzugt 80°C bis 120°C, durchgeführt.

Im zweiten Verfahrensschritt werden als organische Verbindungen (5), die pro Molekül mindestens zwei Isocyanatgruppen aufweisen, bevorzugt solche der allgemeinen Formel

$$O=C=N-R^3-N=C=O \qquad (V),$$

wobei $R^3$ einen zweiwertigen Kohlenwasserstoffrest mit 4 bis 40 Kohlenstoffatomen je Rest bedeutet, eingesetzt.

Beispiele für organische Verbindungen (4) sind Hexamethylen-1,6-diisocyanat, Isophorondiisocyanat, Tolylen-2,4-diisocyanat, Tolylen-2,6-diisocyanat, Phenylen-1,3-diisocyanat, 4,4'-Methylen-bis(cyclohexylisocyanat), 4,4'-Methylen-bis(phenylisocyanat) und Dimethylphenyldiisocyanat.

Organische Verbindungen (4) werden im zweiten Verfahrensschritt vorzugsweise in Mengen von 0,5 bis 1,0 Mol, bevorzugt 0,8 bis 1,0 Mol, Isocyanatgruppe je Mol H-$A^1$-Gruppe im Zwischenprodukt (3) eingesetzt.

In den eingangs erwähnten US 2003/0032726 und US 2003/0032751 wird Polyisocyanat immer im deutlichen Überschuss verwendet, im Gegensatz zu dem erfindungsgemäßen Verfahren. Von geringeren Mengen wird in den eingangs erwähnten US-Schriften abgeraten, da sie die Viskosität des Produktes erhöhen, was damit schwierig zu handhaben ist und ein Lösungsmittel erforderlich macht. Es herrscht damit ein deutliches Vorurteil gegen die erfindungsgemäße Verwendung des Polyisocyanates (4) im Unterschuss von 0,5 bis 1,0 Mol.

Für die Umsetzung im zweiten Schritt des erfindungsgemäßen Verfahrens werden vorzugsweise Kondensationskatalysatoren (6), wie Di-n-butylzinndilaurat, Zinn-II-octoat, Dibutylzinndiacetat, Kaliumoctoat, Zinkdilaurat, Wismuttrilaurat oder tert. Amine, wie Dimethylcyclohexylamin, Dimethylaminopropyldipropanolamin, Pentamethyldipropylentriamin, N-Methylimidazol oder N-Ethylmorpholin, eingesetzt.

Ein bevorzugtes Siloxancopolymer wird erhalten, indem im ersten Verfahrensschritt ein $\alpha,\omega$-Dihydrogenpolydiorganosiloxan (1) im Überschuss mit einem Polyether (2) der Formel (IV) umgesetzt wird und im zweiten Verfahrensschritt das Zwischenprodukt (3), ein HO-Polyether-Polysiloxan-Polyether-OH, mit einem Diisocyanat (4) der Formel (V) umgesetzt wird, wobei UrethanGruppen in das Siloxancopolymer eingeführt werden. Auch freier Polyether aus dem 1. Schritt wird dabei durch Urethanbildung gebunden:

$$CH_2=CH-R^2-(OC_nH_{2n})_m-OC(O)NH-R^3-NHC(O)O[(C_nH_{2n}O)_m-R^2-CH_2CH_2-R_2SiO(R_2SiO)_o- \quad R_2SiO-$$
$$CH_2CH_2-R^2-(OC_nH_{2n})_m-OC(O)NH-R^3-NHC(O)O]_x(C_nH_{2n}O)_m-R^2-CH=CH_2 \qquad (VI),$$

wobei $R$, $R^2$, $R^3$, $n$, $m$ und $o$ die oben dafür angegebene Bedeutung haben und
$x$ 0 oder eine ganze Zahl von 1 bis 20, vorzugsweise 0 oder eine ganze Zahl von 1 bis 4 ist.

Die Urethangruppen in den erfindungsgemäßen hydrophilen Siloxancopolymeren können als Donoren und Akzeptoren bei der Ausbildung von Wasserstoffbrücken wirken.

Im zweiten Schritt des erfindungsgemäßen Verfahrens können zusätzlich zu den organischen Verbindungen (4) noch weitere Verbindungen (7), die gegenüber Isocyanatgruppen reaktiv sind, eingesetzt werden. Beispiele für weitere Verbindungen (7) sind solche ausgewählt aus der Gruppe der Formeln

$$R^4- (A-C_nH_{2n})_m-A^1-H \qquad (VII),$$

$$HO-R^5-NR^4-R^5-OH \qquad (VIII),$$

$$HO-R^5-NR^4_2 \qquad (IX),$$

$$HO-R^6(NR^4_2)_2 \qquad (X),$$

$$HO-R^7(NR^4_2)_3 \qquad (XI),$$

$$(HO)_2R^6-NR^4_2 \qquad (XII),$$

$$HNR^4_2 \qquad (XIII)$$

wobei $R^4$ ein Wasserstoffatom oder einen Rest R, der gegebenenfalls ein oder mehrere Stickstoffatome enthalten kann, bedeutet,

$R^5$ einen zweiwertigen Kohlenwasserstoffrest mit 1 bis 10 Kohlenstoffatomen je Rest bedeutet,

$R^6$ einen dreiwertigen organischen Rest mit 1 bis 100 Kohlenstoffatomen je Rest, vorzugsweise einen dreiwertigen Kohlenwasserstoffrest mit 1 bis 100 Kohlenstoffatomen, der ein oder mehrere Sauerstoffatome enthält,

$R^7$ einen vierwertigen organischen Rest mit 1 bis 100 Kohlenstoffatomen je Rest, vorzugsweise einen vierwertigen Kohlenwasserstoffrest mit 1 bis 100 Kohlenstoffatomen, der ein oder mehrere Sauerstoffatome enthält, und

$A^1$, n und m die oben dafür angegebene Bedeutung haben.

Beispiele für Verbindungen der Formel (VII) sind Methylpolyethylenoxid, Butylpolyethylenoxid, Methylpolyethylenoxid/polypropylenoxid und Methylpolypropylenoxid.

Beispiele für Verbindungen der Formel (VIII) sind N-Methyldiethanolamin, N-Methyldipropanolamin, Dimethylaminopropyldipropanolamin, N-Dodecyldiethanolamin und N-Stearyldipropanolamin.

Beispiele für Verbindungen der Formel (IX) sind N,N-Dimethylethanolamin, N,N-Diethylpropanolamin, N,N-Dimethylaminopropyl-methylethanolamin und Dimethyl-2-(2-aminoethoxy)ethanol.

Beispiele für Verbindungen der Formel (X) sind 1,5-Bis(dimethylamino)-pentan-3-ol, 1,5-Bis(methylamino)-pentan-3-ol, 1,7-Bis(dimethylamino)-heptan-4-ol und N,N-Bis-(3-dimethylaminopropyl)-N-isopropanolamin

Beispiele für Verbindungen der Formel (XI) sind 2,4,6-Tris(dimethylaminomethyl)-phenol, 1,1,1-Tris(dimethylaminomethyl)-methanol und 2,4,6- Tris(dimethylaminomethyl)-cyclohexanol.

Beispiele für Verbindungen der Formel (XII) sind N,N-Bis(dimethylaminopropyl)-3-aminopropan-1,2-diol, N,N-Bis(dimethylaminopropyl)-2-aminopropan-1,3-diol, N,N-Bis(3-dimethylaminopropyl)-carbaminosäuremonoglycerid,

Beispiele für Verbindungen der Formel (XIII) sind Dibutylamin, Octylamin, Benzylamin, 3-(Cyclohexylamino)-propylamin, 2-(Diethylamino)-ethylamin, Dipropylentriamin, Isophorondiamin, Dimethylaminopropylmethylamin, Aminopropylmorpholin, N,N-Bis(dimethylaminopropyl)amin, Dimethylaminopropylamin.

Verbindungen der Formel (VIII) bis (XIII) bieten die Möglichkeit in dem Siloxancopolymer protonierbaren Stickstoff einzubauen.

Verbindungen der Formel (VII) werden im zweiten Verfahrensschritt in Mengen von vorzugsweise 0 bis 2 Mol, bevorzugt 0 bis 1 Mol, H-$A^1$-Gruppe je Mol H-$A^1$-Gruppe in Verbindung (2) eingesetzt.

Verbindungen der Formel (VIII) werden im zweiten Verfahrensschritt in Mengen von vorzugsweise 0 bis 2 Mol, bevorzugt 0 bis 1 Mol, HO-Gruppe je Mol H-$A^1$-Gruppe in Verbindung (2) eingesetzt.

Verbindungen der Formel (IX) werden im zweiten Verfahrensschritt in Mengen von vorzugsweise 0 bis 2 Mol, bevorzugt 0 bis 1 Mol, HO-Gruppe je Mol H-$A^1$-Gruppe in Verbindung (2) eingesetzt.

Verbindungen der Formel (X) werden im zweiten Verfahrensschritt in Mengen von vorzugsweise 0 bis 2 Mol, bevorzugt 0 bis 1 Mol, HO-Gruppe je Mol H-$A^1$-Gruppe in Verbindung (2) eingesetzt.

Verbindungen der Formel (XI) werden im zweiten Verfahrensschritt in Mengen von vorzugsweise 0 bis 2 Mol, bevorzugt 0 bis 1 Mol, HO-Gruppe je Mol H-$A^1$-Gruppe in Verbindung (2) eingesetzt.

Verbindungen der Formel (XII) werden im zweiten Verfahrensschritt in Mengen von vorzugsweise 0 bis 2 Mol, bevorzugt 0 bis 1 Mol, HO-Gruppe je Mol H-$A^1$-Gruppe in Verbindung (2) eingesetzt.

Verbindungen der Formel (XIII) werden im zweiten Verfahrensschritt in Mengen von vorzugsweise 0 bis 2 Mol, bevorzugt 0 bis 1 Mol, HN-Gruppe je Mol H-$A^1$-Gruppe in Verbindung (2) eingesetzt.

Auch bei der Verwendung von Verbindungen (7) wird Polyisocyanat (5) vorzugsweise im Unterschuss eingesetzt, um ein sicheres Abreagieren der gesundheitsgefährdenden Isocyanatgruppen zu gewährleisten. Organische Verbindungen (4) werden daher im zweiten Verfahrensschritt vorzugsweise in Mengen von 0,5 bis 1,0 Mol, bevorzugt 0,8 bis 1,0 Mol, Isocyanatgruppe je Mol der Summe mit Isocyanatgruppen reagierender Funktionen aus der Summe von Zwischenprodukt (3) und Verbindungen (7) eingesetzt.

Der zweite Verfahrensschritt wird vorzugsweise beim Druck der umgebenden Atmosphäre, also etwa bei 1020 hPa (abs.), durchgeführt, es kann aber auch bei höheren oder niedrigeren Drücken durchgeführt werden. Ferner wird der zweite Verfahrensschritt vorzugsweise bei einer Temperatur von 40°C bis 140°C, bevorzugt 60°C bis 100°C, durchgeführt.

Zur Erniedrigung der teilweise sehr hohen Produktviskositäten können gegebenenfalls niedermolekulare Stoffe, wie Alkohole oder Ether zugesetzt werden. Beispiele hierfür sind Ethanol, Isopropanol, n-Butanol, 2-Butoxyethanol, Diethylenglycolmonobutylether, Tetrahydrofuran, Diethylenglycoldiethylether und Dimethoxyethan, wobei Diethylenglycolmonobutylether ein bevorzugtes Beispiel ist. Bevorzugte Zusatzmengen sind im Fall sehr viskoser Produkte bis zu 50 Gew.-%, besonders bevorzugt bis zu 30 Gew.-%, bezogen auf die erfindungsgemäßen hydrophilen Siloxancopolymere. Derartige Zusätze haben außerdem den Vorteil, dass die daraus entstehenden Produkte in Wasser leichter dispergierbar sind als die reinen Siloxancopolymere.

Die erfindungsgemäßen Siloxancopolymere können leicht in Wasser ohne weitere Hilfsstoffe, wie Emulgatoren, dispergiert werden, sind damit selbstdispergierend, und ergeben Emulsionen, insbesondere Microemulsionen.

Bei dem erfindungsgemäßen Verfahren zur Modifizierung faserartiger Substrate werden die erfindungsgemäßen Siloxancopolymere vorzugsweise in Form ihrer wässrigen Emulsionen, bevorzugt wässrigen Microemulsionen, enthaltend

(A) erfindungsgemäße Siloxancopolymere und
(B) Wasser,

eingesetzt.

Die Emulsion enthält vorzugsweise 20 bis 60, bevorzugt 30 bis 50 Gew.-% der erfindungsgemäßen Siloxancopolymere (A).

Die erfindungsgemäßen Emulsionen, bevorzugt Microemulsionen, werden durch Vermischen von

(A) erfindungsgemäßen Siloxancopolymeren mit
(B) Wasser

hergestellt.

Technologien zur Herstellung von Siliconemulsionen sind bekannt. Üblicherweise erfolgt die Herstellung durch einfaches Verrühren der erfindungsgemäßen Siloxancopolymere mit Wasser und gegebenenfalls anschließendes Homogenisieren mit Rotor-Stator-Homogenisatoren, Kolloidmühlen oder Hochdruckhomogenisatoren.

Die erfindungsgemäßen Siloxancopolymere oder deren Emulsionen können verwendet werden im Haushaltsbereich als hydrophile Weichspüler, in der Kosmetikindustrie als Bestandteil von Haarshampoo-, Haarpflege- und Conditionerformulierungen, im Textilbereich zur Ausrüstung von Geweben, Fasern oder auch Leder mit hydrophilen Weichmachern, sowie als hydrophile Weichmacher für Non-wovens. Die einzelnen Anwendungsarten sind nachfolgend näher beschrieben.

Das Waschen und Reinigen von Wäsche in wässrigen Waschflotten ist ein komplexer Prozess, der das Zusammenwirken zahlreicher physikalischer und chemischer Einflüsse erfordert. Ganz allgemein kann Waschen definiert werden sowohl als das Entfernen von schwer löslichen Materialien von textilen Oberflächen durch Wasser oder Tensidlösungen sowie auch das Weglösen von wasser-löslichen Verunreinigungen von solchen Oberflächen.

Durch das Waschen von Textilien in der Waschmaschine werden diese mechanisch wesentlich stärker belastet als durch die Handwäsche. So kann die in der Waschmaschine gewaschene Wäsche so stark gepresst werden, dass die Faserstapel auf der Textiloberfläche in einen Zustand starker Unordnung gebracht wird, vor allem wenn es sich um Naturfasern wie Baumwolle oder Wolle handelt. Durch die wiederholte Trocknung in unbewegter Luft (v.a. wenn Wäsche zum Trocknen in Räumen aufgehängt wird), werden diese Bedingungen in den Geweben fixiert und die Wäsche nimmt einen harten Griff an.

Neben der Weichmachung sind auch andere Effekte, die die Wäschepflege und den Tragekomfort erhöhen, wünschenswert. Dazu gehört etwa die Erleichterung beim Bügeln durch Herabsetzung der Reibung zwischen Bügeleisen und Textiloberfläche. Auch die Reduzierung von Knittern, die weniger Bügelarbeit erforderlich macht sowie der Schutz vor dem Zerknittern sind gewünschte Effekte. Solche Zusatzeffekte sollten allerdings möglichst ohne Reduzierung der Hydrophilie der Textilien erreicht werden.

Zur Erreichung besagter Effekte werden Aktivkomponenten in Form von Flüssigweichspülern beim letzten Spülgang zugesetzt. So können z.B. kationische Tenside auf Basis quaternärer Ammoniumverbindungen den Weichgriff verbessern.

Ein Anliegen der Waschmittelhersteller wäre es natürlich auch die beschriebenen Zusatzeffekte bereits beim eigentlichen Waschprozess einzuführen in Form von sog. "2 in 1" Produkten. Allerdings ist es für einen erfolgreichen Einsatz solcher Produkte notwendig die Konkurrenz, die beim Waschprozess hinsichtlich der Desorption von Verunreinigung und dem für textile Effekte notwendige Adsorption von Aktivkomponenten entgegenzuwirken. In dieser Richtung sind bisher noch keine den Endverbraucher zufrieden stellenden vermarktungsfähigen Produkte entwickelt worden. Die erfindungsgemäßen Siloxancopolymere lösen dieses Problem.

Die erfindungsgemässen Siloxancopolymere können, in Abhängigkeit von der gewählten Stöchiometrie wasserlöslich oder selbstemulgierend (sog. "Selbstemulgierende Systeme") eingestellt werden, d.h. sie erfordern für die Emulgierung keine weiteren Hilfsmittel. Die Siloxancopolymere können zur Behandlung von textilen Flächengebilden, Textilfasern und Leder, als Additive in Beschichtungen und Lacken, als Zusätze in kosmetischen Formulierungen und als oberflächenaktive Mittel verwendet werden. Sie weisen insbesondere hervorragende Eigenschaften als Textilweichmacher auf, die denen gewöhnlicher Amino-Glykolöle überlegen sind.

Auf Grund ihrer Kationogenität und polarität welche durch die Anzahl der Amino-, Carbamid- und Harnstoffgrppen im Molekül bedingt ist, haften die erfindungsgemäßen Copolymere sehr gut auf Substraten wie Textilien oder Papier und zeichnen sich durch ihre für Organosiliciumverbindungen vergleichsweise hohe Hydrophilie bei gleichzeitig herausragender Griffverbesserung aus. Im Vergleich zu dem Stand der Technik entsprechenden auf Aminofunktionellen, Glykolfunktionellen, Amidofunktionellen und Aminoglykolfunktionellen Appreturprodukten zeichnen sich die erfindungsgemäßen Copolymere durch verbesserte Ausziehfähigkeit, Permanenz gegenüber Wäschen und chemischer Reinigung, verbesserter Verklebbarkeit, Stabilität gegenüber Scherkräften und pH-Änderungen und die Möglichkeit synergistische Formulierungen herstellen zu können.

Die Siloxancopolymere können daher z. B. als Bestandteile von Emulsionen, in Lösung oder in Substanz der Behandlung von textilen Flächengebilden, wie z. B. Geweben, Maschenwaren oder fliesen, der Textilfaser- und Garnpräparation und -modifikation sowie der Leder- und Papierbehandlung dienen. Durch die Ausrüstung oder Modifikation mit den entsprechenden Siloxancopolymeren können gewünschte Eigenschaften wie z.B. ein weicher, fließender Griff, verbesserte Elastizität, antistatische Eigenschaften, Farbvertiefung, Reibwerte, Oberflächenglätte, Glanz, Knittererholung, Farbechtheiten, Waschbeständigkeit, Hydrophilie, Weiterreißfestigkeit, verringerte Pillingneigung, "Easy-Care"- und "Soil-Release"-Eigenschaften, sowie verbesserter Tragekomfort verliehen werden. Die durch die Ausrüstung mit den Siloxancopolymeren erzielten Effekte weisen, je nach Struktur der Siloxancopolymere, des Substrates und den Waschbedingungen, eine gute bis sehr gute Beständigkeit gegenüber Wasch- und Pflegeprozessen auf.

Weiterhin können durch die Ausrüstung oder Modifikation von textilen Flächengebilden, Fasern, Garnen, Papier und Leder mit den Siloxancopolymeren die industrielle Verarbeitbarkeit, wie z.B. die Verarbeitungs- und Produktionsgeschwindigkeit, Korrekturmöglichkeiten sowie die Qualität der Materialien verbessert werden.

Die textilen Flächengebilde, Fasern und Garne können gefertigt sein aus Mineralfasern, wie Glasfasern oder Silikatfasern, Naturfasern wie z.B. Wolle, Seide oder Baumwolle, Kunstfasern, wie z.B. Polyester- oder Polyamidfasern, Cellulosefasern, Mischpolymerfasern oder Metallfasern. Filamentfasern oder Stapelfasern aus den genannten Substraten können ebenfalls eingesetzt werden. Des weiteren können auch Flächengebilde aus Fasermischungen, wie z.B. Baumwolle/Polyester, Papier sowie natürliche Flächengebilde, wie Leder, verwendet werden.

Die Beschichtung oder Ausrüstung kann im Rakelverfahren, Tauch (Quetsch) (Dip)-Verfahren, Extrusionsverfahren, Spritz-Beflockungs- oder Sprühverfahren, Foulard-, Auszieh- oder Tauch-Schleuder-Verfahren aufgebracht werden. Auch sind alle Arten von Rollenbeschichtungen, wie Gravurwalzen, Pflatschen oder Auftrag über Mehrwalzensysteme, sowie Druck, z.B. (Rotary) Siebdruck, möglich. Des weiteren kann die Ausrüstung oder Beschichtung durch Schaumauftrag und nachträgliches Kalandrieren, mit einem Kalander oder Schmelzkalander erfolgen.

Des weiteren können die Siloxancopolymere als Additive in Beschichtungen, Lacken und Lasuren Einsatz finden. Zusätze der Siloxancopolymere zu z. B. strahlungs- oder additionshärtenden Lacken führen zur Verringerung der Oberflächenrauhigkeit und somit zu einer Verringerung des Gleitwiderstands des Lacks.

Weiterhin können die Siloxancopolymere als Zusätze in kosmetischen Formulierungen, beispielsweise als Konditionierer in Haarwaschmitteln, und als Bautenschutzmittel dienen.

Zusätzlich stellen die Siloxancopolymere oberflächenaktive Mittel dar und können als Detergentien, Tenside, Emulgatoren, Entschäumer und Schaumstabilisatoren eingesetzt werden.

**Herstellung der erfindungsgemäßen Siloxancopolymere und deren wässrige Emulsionen:**

Beispiel 1:

491 g eines $\alpha,\omega$-Dihydrogenpolydimethylsiloxans mit 0,055 Gew.-% Si-gebundenem Wasserstoff und einem Wassergehalt von 50 Gew.-ppm werden mit 1001 g eines Allyalkoholethoxylat/propyloxylats der Formel

$$H_2C=CH-CH_2- (OCH_2CH_2)_a[OCH_2CH(CH_3)]_b-OH,$$

mit einem Verhältnis a : b = 1,0, einem Wassergehalt von 978 Gew.-ppm und einer Jodzahl von 13,7 (als Jodzahl wird die Zahl bezeichnet, welche die bei der Addition an die aliphatische Mehrfachbindung verbrauchte Jodmenge in Gramm pro 100 Gramm eingesetztes, zu untersuchendes Material angibt) vermischt. Die Mischung wird auf 100°C erwärmt und zur Mischung werden 0,28 g einer 2,7 Gew.-%igen (bezogen auf elementares Platin) Lösung eines Platin-1,3-Divinyl-1,1,3,3-tetramethyldisiloxan-Komplexes in einem $\alpha,\omega$-Divinyldimethylpolysiloxan mit einer Viskosität von 1000 mPa·s bei 25°C, eine Lösung des sogenannten Karstedt-Katalysators (dessen Herstellung in US 3,775,452 beschrieben ist), zudosiert. Das Reaktionsgemisch erwärmt sich um ca. 6°C, worauf die gleiche Menge Katalysator nachdosiert wird. Das Reaktionsgemisch wird daraufhin homogen. Nach einer Stunde Reaktionszeit bei 100 bis 110°C wird eine Probe des Polyether-Polysiloxan-Zwischenproduktes abgekühlt, das eine Viskosität von 2 220 mm$^2$/s bei 25°C aufweist.

Bei 100°C werden nun 45,5 g Hexamethylen-1,6-diisocyanat (1,0 Mol Isocyanatgruppe je Mol HO-Gruppe des Zwischenproduktes) zudosiert und die Urethanbildung wird mit 100 mg Di-n-butylzinndilaurat katalysiert. Nach zwei Stunden bei 100°C wird das klare Reaktionsprodukt abgekühlt. Es besitzt eine Viskosität von ca. 100 000 mPa·s bei 25°C.

40 g des hochviskosen Öls werden bei 50°C mit 60 g Wasser vermischt. Das Produkt ist leicht emulgierbar und bildet eine opaleszierende Microemulsion mit einem Urethangehalt von 0,14 mequ./g.

Vergleichsversuch:

Beispiel 1 wird sinngemäß wiederholt, indem aber zum Vergleich eine andere Charge des Polyethers eingesetzt wird, die produktionsbedingt 3.620 ppm Wasser enthält. Auf den gesamten Ansatz bezogen, beträgt der Wassergehalt statt 636 ppm nun 2.350 ppm Wasser.

Bei der Umsetzung mit Hexamethylen-1,6-diisocyanat entsteht eine starke Schaumbildung. Nach Reaktionsende erhält man ein kaum noch rührbares Öl, das nach Einarbeitung der 1,5-fachen Menge Wasser (40% Ölgehalt) spontan keine Emulsion bildet. Langes Einbringen starker Scherkräfte mit einem Turraxgerät bildet ein trübes, inhomogenes Gemisch.

Beispiel 2:

960 g des $\alpha,\omega$-Dihydrogenpolydimethylsiloxans mit einem Wassergehalt von 50 Gew.-ppm aus Beispiel 1 werden mit 536 g eines Polyethers der Formel

$$H_2C=CH-CH_2-(OCH_2CH_2)_{10,2}-OH ,$$

mit einem Wassergehalt von 686 Gew.-ppm gemischt und auf 100°C erwärmt. Dann werden 0,28 g der in Beispiel 1 beschriebenen Karstedt-Katalysatorlösung zugegeben, worauf die Temperatur des Reaktionsgemisches auf 19 °C ansteigt und ein klares Produkt entsteht. Nach einer Stunde bei 100 bis 110°C wird vollständiger Umsatz des Si-gebundenen Wasserstoffs erreicht. Das Polyether-Polysiloxan-Zwischenprodukt hat eine Viskosität von 760 mm$^2$/s bei 25°C.

Es werden nun 63 g N-Methyldiethanolamin (1,02 Mol HO-Gruppe je Mol HO-Gruppe des Polyethers)und 178 g Hexamethylendiisocyanat (0,99 Mol Isocyanatgruppe je Mol der Summe der HO-Gruppen des Zwischenproduktes und des N-Methyldiethanolamins) nacheinander zudosiert. Die Urethanbildung wird mit 100 mg Di-n-butylzinndilaurat katalysiert. Nach zwei Stunden bei 100°C wird abgekühlt und es werden bei 70°C 64 g Essigsäure zugegeben. Das klare, bräunliche Produkt besitzt eine Viskosität von 120 000 mPa·s bei 25°C.

40 g des hochviskosen Öls werden bei 50°C mit 60 g Wasser vermischt. Unter leichtem Rühren bildet sich eine Microemulsion mit einem Urethangehalt von 0,39 mequ./g und einer Aminzahl von 0,12 (Aminzahl entspricht der Anzahl von ml 1N HCl, die zur Neutralisation von 1 g Substanz erforderlich ist).

Beispiel 3:

1411 g des Allylalkoholethoxylats/propoxylats aus Beispiel 1 werden mit 813 g eines $\alpha,\omega$-Dihydrogenpolydimethylsiloxans mit 0,052 Gew.-% Si-gebundenem Wasserstoff vermischt und gut gerührt auf 100°C erwärmt. Nach identischer Katalyse erhält man nach einer Stunde Reaktionszeit ein Polyether-Polysiloxan-Zwischenprodukt mit einer Viskosität von 2.490 mm$^2$/s bei 25°C.

Bei 100 °C werden 83 g N,N-Bis(3-dimethylaminopropyl-)N-isopropanolamin eingerührt und 92 g Hexamethylendiisocyanat zudosiert. Der Quotient aus NCO-Gruppen zu der Summe der damit reagierenden organischen Gruppen beträgt 0,995, unter Berücksichtigung des darin enthaltenen Wassers nur 0,87. Nach schwach exothermer Reaktion erwärmt man auf 120°C und lässt nach Zugabe von 50 mg Dibutylzinnlaurat bei gleichzeitigem Anstieg der Viskosität weitere 3 Stunden ausreagieren, bis im IR kein Isocyanat mehr nachgewiesen werden kann. Das bei 25°C sehr viskose Öl hat einen Gehalt an basischem Stickstoff von 0,42 mequ./g.

Beispiel 4:

635 g des $\alpha,\omega$-Dihydrogenpolydimethylsiloxans aus Beispiel 3 werden mit 205 g eines Polyethers der Formel

$$H_2C=CH-CH_2-(OCH_2CH_2)_{9,5}-OH,$$

wie in Beispiel 2 umgesetzt. Das Polyether-Polysiloxan-Zwischenprodukt hat eine OH-Konzentration von 0,512 mequ./g und enthält 177 ppm Wasser.

200 g dieses Zwischenprodukts werden mit 10,3 g Bis-(dimethylaminopropyl)amin gemischt und auf 84°C erwärmt; 13,2 g Hexamethylendiisocyanat werden zudosiert.

Das Verhältnis der NCO-Gruppen zur Summe der damit reagierenden organischen Funktionen beträgt 0,998, bei Einbeziehung von Wasser 0,97.

Ohne weitere Katalyse wird in einer Stunde bei ca. 90°C in leicht exothermer Reaktion kompletter Umsatz der Isocyanatgruppen erreicht. Das Polymergemisch enthält 0,49 mequ. basischen Stickstoff pro Gramm.

32 g dieses Polymers werden mit einer Lösung von 1,04 g Essigsäure in 8 g Diethylenglycolmonobutylether, neutralisiert. Mit 60 g Wasser bildet sich nach Verrühren mit einem Spatel spontan eine schwach gelbliche Mikroemulsion.

Beispiel 5:

200 g des in Beispiel 4 hergestellten Polyether-Polysiloxanzwischenproduktes (0,512 mequ. OH/g) werden mit zusätzlichen 26,2 g des zu seiner Synthese verwendeten Polyethers sowie 14,8 g Bis-(dimethylaminopropyl)amin versetzt und auf 80°C erwärmt. Die Zugabe von 19,8 g Hexamethylendiisocyanat startet sofort eine mäßig exotherme Reaktion, die nach ca. 2 Stunden bei 90°C beendet ist, und kein Isocyanat mehr nachgewiesen werden kann. Das Verhältnis von NCO zur Summe der damit reagierenden Gruppen (OH, NH) beträgt ohne die Einbeziehung von Wasser bereits 0,995; unter Berücksichtigung des enthaltenen Wassers nur 0,97.
Das hochviskose Polymergemisch hat eine Konzentration von basischem Stickstoff von 0,60 mequ./g.
Zur Herstellung einer Mikroemulsion werden 32 g dieses Produkts mit einer Lösung von 1,29 g Essigsäure in 8 g Diethylenglycolmonobutylether neutralisiert und danach mit 60 g Wasser verrührt.

Beispiel 6:

200 g des in Beispiel 4 hergestellten Polyether-Polysiloxan-Zwischenproduktes (0,512 mequ. OH/g) werden nur mit 4,5 g Bis-(dimethylaminopropyl)amin ohne weitere Polyetherzusätze auf 88°C erwärmt. Die Zugabe von 10,6 g Hexamethylendiisocyanat startet eine leicht exotherme Reaktion. Das Verhältnis von NCO-Gruppen zur Summe der damit reagierenden organischen Funktionen beträgt 0,998, bei Berücksichtigung des im Reaktionsgemisch enthaltenen Wassers 0,97.
Nach 1 Stunde bei 100°C ist kein Isocyanat mehr nachweisbar. Das hochviskose Polymer hat einen Gehalt an basischem Stickstoff von 0,22 mequ./g.
Nach Neutralisation von 32 g basischem Produkt mit einer Lösung aus 0,46 g Essigsäure in 8 g Diethylenglycolmonotbutylether erhält man durch Einrühren von 60 g Wasser eine stabile Mikroemulsion.

Beispiel 7

Beispiel 2 wird sinngemäss wiederholt, wobei in der zweiten Stufe das N-Methyldiethanolamin durch 99g Bis-(dimethylaminopropyl)-amin ersetzt wird. Die Menge Hexamethylendiisocyanat wird auf 131g reduziert (0,98 Mol Isocyanat je Mol der Summe aus damit reaktiven OH- und NH-Gruppen). Nach komplettem Umsatz aller Isocyanatgruppen wird mit 70g Essigsäure neutralisiert und mit 450g Diethylenglycolmonobutylether verdünnt. Bei einem Polymergehalt von 80% hat diese Lösung eine Viskosität von 4900 mm$^2$/s bei 25 °C und eine Aminzahl von 0,47.
In 40 g dieser Lösung werden bei Raumtemperatur portionsweise insgesamt 60 g Wasser eingerührt, wobei sich eine feinteilige Emulsion mit einer Aminzahl von 0,19 bildet.

Beispiel 8

In diesem Beispiel werden gegenüber Beispiel 7 reduzierte Mengen an gegenüber Isocyanat monofunktionellen Rohstoffen eingesetzt: Der Polyether wird von 536 g auf 402 g und das Amin von 99 g auf 50 g reduziert. Damit enthält die Reaktionsmischung 1,06 Mol mit Isocyanat reaktive Gruppen, wodurch sich die Menge von Hexamethylendiisocyanat auf 87 g reduziert. Die Neutralisation erfolgt mit 35 g Essigsäure. Durch Verdünnung mit 384 g Diethylenglycolmonobutylether erhält man eine klare 80%ige Amino-PUR-Siliconpolyetherlösung mit 5100 mm$^2$/s (25°C) und einer Aminzahl von 0,28.
Auch diese Lösung wird analog Beispiel 7 emulgiert. Die gebildete feinteilige Emulsion hat eine Aminzahl von 0,113.

Beispiel 9

960 g des $\alpha$, $\omega$-Dihydrogenpolydimethylsiloxans aus Beispiel 1 werden mit 125 g eines Polyethers der Formel

$$H_2C=CH-CH_2\text{-} (OCH_2CH_2)_{3,0}\text{-}OH$$

mit einem von 780 Gew.-ppm wie dort zur Reaktion gebracht. Nach vollständigem Umsatz des Si-gebundenen Wasserstoffs wird das Produkt bei 140°C im Vakuum ausgeheizt, wonach man 1060 g eines klaren $\alpha$, $\omega$-Dihydroxysiloxancopolymers erhält. Hierzu werden bei 100°C nacheinander 70 g Bis(dimethylaminopropyl)amin und 74g Hexamethylendiisocyanat dosiert. Nach zwei Stunden bei 100°C sind alle NCO-Gruppen umgesetzt, und man neutralisiert mit 49 g Essigsäure und verdünnt aus Gründen einfacherer Handhabung mit 313 g Diethylenglycolmonobutylether. Die 80%ige Formulierung hat eine Viskosität von 2200 mm$^2$/s (25°C) und eine Aminzahl von 0,35.

Die Emulgierung analog Beispiel 7 liefert eine feinteilige Emulsion mit der Aminzahl 0,14.

Beispiel 10

1492 g des Polyether-Polysiloxan-Zwischenproduktes von Beispiel 1 werden bei 100°C mit 51 g Bis(dimethylaminopropyl)-amin und 67 g Hexamethylendiisocyanat vermischt. Die leicht exotherme Reaktion ergibt nach zwei Stunden vollständigen Umsatz der NCO-Gruppen. Neutralisation mit 35 g Essigsäure und weitere Verdünnung mit 410 g Diethylenglycolmonobutylether liefert eine klare Formulierung mit einer Viskosität von 7800 mm$^2$/s (25°C) und einer Aminzahl von 0,26.

In 40 g dieser Verdünnung lassen sich mühelos 60 g Wasser einrühren. Die wässrige Formulierung hat eine Aminzahl von 0,104.

**Verwendung der erfindungsgemäßen Siloxancopolymere:**

Beispiel 11: Weichspüleranwendung

Je 8 Stück Frotteehandtücher (225 g), 8 glatte Baumwollgewebe (20 x 160 cm, 50 g) und 8 glatte Mischgewebe (15 x 100 cm, 45 g) werden zweimal mit 130,0 g silikonfreiem Pulvervollwaschmittel im Vollwaschgang bei 95°C ausgewaschen. Danach werden die Gewebe noch zweimal durch Starten des Spülgangs gespült. Zur Behandlung der Stoffe mit den erfindungsgemäßen Siloxancopolymeren gibt man je ein Frotteehandtuch, ein glattes Baumwollgewebe und ein glattes Mischgewebe in die Waschmaschine und startet im 1. Spülgang mit vollentsalztem Wasser. Beim Start des 3. und letzten Spülgangs öffnet man das Bullauge der Waschmaschine und gibt in die Trommel 1,5 Trinkwasser für eine resultierende Wasserhärte von 3°dH, sowie 5 g Eisessig für einen pH-Wert von 4. Dann werden jeweils 10,16 g erfindungsgemäße Siliconemulsion gemäß den Beispielen 7-9, entsprechend 1,0% Siliconwirkstoff, bezogen auf das Textilgewicht, in die Trommel gegeben und der letzte Spülgang gestartet. Nach dem Lufttrocknen der Gewebe und Klimatisieren bei 23°C und 60% relativer Luftfeuchte über Nacht erfolgen die anwendungstechnischen Prüfungen.
Die Bestimmung des Weichgriffs erfolgt in einem Panel Test. Dazu nummeriert man die Frotteehandtücher von 1 bis n durch. Eine Versuchsperson lässt man in einem Blindtest Handtuch 1 mit 2 vergleichen. Ist Handtuch 1 weicher erhält es die Note 1, ist es härter Note 0 und sind beide gleich 0,5. Dann werden Handtuch 1 mit 3, 1 mit 4, usw. verglichen, bis zum Vergleich von Handtuch n-1 mit n. Als Ergebnis werden alle Benotungen eines Handtuchs zusammengezählt. Der Test ist mit mindestens 3 Testpersonen durchzuführen.
Beim Bügeltest werden die glatten Baumwoll- und Mischgewebe auf Stufe "Baumwolle" ohne Dampf gebügelt und man bestimmt die Anzahl der Bügelgänge über eine Gewebestelle, die notwendig sind um das Gewebe knitterfrei zu bügeln.
Beim Rewetting-Test gibt man auf das Gewebe einen Tropfen blaues VE-Wasser aus 1 cm Höhe und stoppt die Zeit bis der Tropfen soweit aufgesaugt wurde, dass an der benetzten Stelle
die ersten Gewebestrukturen erkennbar sind.
Beim Rutschtest lässt man ein auf Stufe "Baumwolle" erhitztes Bügeleisen ohne Dampf bei einem Winkel von 6° das Gewebe herabgleiten. Auf einer Strecke von 90 cm stoppt man die benötigte Rutschzeit.
Die Ergebnisse der anwendungstechnischen Prüfungen sind in Tabelle 1 zusammengefasst.

Tabelle 1:

| Silicon-emulsion aus | Weichgriff (Frottee) | Rewetting (Frottee) | Rutschtest (Baumwolle) | Rutschtest (Mischgewebe) |
|---|---|---|---|---|
| Blindwert (unbehandelt) | 0 | 1 s | >60 s | 10 s |
| Beispiel 8 | 1,7 | 2 s | 10 s | 8 s |
| Beispiel 7 | 1,0 | 1 s | 15 s | 10 s |
| Beispiel 9 | 3,0 | 1 s | 5 s | 4 s |

Gegenüber den unbehandelten Frottehandtüchern wird der Weichgriff durch die erfindungsgemäßen Siloxancopolymere erheblich verbessert, ohne die Wasseraufnahme merklich zu verschlechtern. Die Rutschtests fallen signifikant besser aus.

Beispiel 12: Textilweichmacheranwendung

Zur Textilausrüstung wurde eine gebleichte, unausgerüstete Webware PES/CO 65/35 Twill mit einem Flächengewicht von 200g/m$^2$ (Stoff 1) sowie unausgerüstete Maschenware 100 % CO Cretone mit einem Flächengewicht von 230 g/m$^2$ (Stoff 2) verwendet. Als Referenz diente eine Ausrüstung mit einer 33 %igen Standard-Silicon-Weichmacheremulsion (Micro-Emulsion eines aminofunktionellen Polydimethylsiloxans = Vergleich) käuflich erwerblich bei der Firma Wacker-Chemie GmbH unter dem Handelsnamen Finish CT 34 E, sowie mit Wasser geklotzte und getrocknete Ware (= Blindversuch).

Der Stoff wurde mit der jeweiligen Flotte getränkt, mit einem Zweiwalzenfoulard auf 70 % Flottenaufnahme abgequetscht, aufgespannt und in einem Mathis-Laborspannrahmen bei 150°C zwei Minuten getrocknet. Anschließend wurde die Ware mindestens 12 Stunden im Klimaraum bei 23°C und 50 % Luftfeuchtigkeit klimatisiert.

Bestimmungsmethoden für die Ergebnisse der Anwendungsbeispiele:

Bestimmung des Weichgriffs (Griffbewertung):

Da der Weichgriff von Textilien stark dem subjektiven Empfinden der Testpersonen unterliegt, kann nur eine Standardisierung der Randbedingungen, nicht aber der Bewertung erreicht werden. Um trotzdem eine Reproduzierbarkeit zu gewährleisten, wurden die ausgerüsteten Muster hinsichtlich ihres Weichgriffs beurteilt und in eine Rangfolge gebracht. Dazu wurden von 10 Personen in Abhängigkeit der Anzahl n der getesteten Muster 1 bis n Punkte vergeben, wobei n Punkte für das weichste Muster und 1 Punkt für das am wenigsten weiche Muster vergeben wurden. Die Griffbewertung eines Musters errechnet sich somit als Mittelwert der jeweils auf dieses Muster entfallenen Punkte.

Bestimmung der Tropfeneinsinkzeit:

Das ausgerüstete Muster wurde nach der Ausrüstung acht Stunden zur Akklimatisation in einem Klimaraum bei einer Temperatur von 23°C und einer Luftfeuchtigkeit von 50 % gelagert, dann wurde ein Tropfen entionisiertes Wasser aus einer Höhe von 6 cm auf die gespannte Stoffoberfläche gegeben und die Zeit bestimmt, nach der der Wassertropfen vom Stoff aufgesaugt war, längstens jedoch drei Minuten. Es wurden fünf Bestimmungen durchgeführt und der Mittelwert gebildet.

In Tabelle 2 sind für einige Anwendungsbeispiele die Ergebnisse des mittels Foulardverfahren ausgerüsteten Stoffs zusammengestellt.

Tabelle 2:

| Verwendungsbeispiele | 12a | 12b | 12c | Blind-versuch (unbeh.) | Vergleich |
|---|---|---|---|---|---|
| Beispiel 7 | 5 g/l | - | 3 g/l | - | - |
| Beispiel 10 | - | 5 g/l | 2 g/l | - | - |
| 33 %ige Standard-Silicon-Micro-Emulsion | | - | - | - | 15 g/l |
| Eisessig | 0,5 g/l | 0,5 g/l | 0,5 g/l | 0,5 g/l | 0,5 g/l |
| Tropfeneinsinkzeit(s)Stoff 1 | 50 | 3 | 15 | 108 | >180 |
| Tropfeneinsinkzeit(s)Stoff 2 | 3 | <1 | 1 | 2 | >180 |
| Griff Stoff 1 | 4,6 | 2,1 | 3,2 | 0 | 4,1 |
| Griff Stoff 2 | 4,5 | 2 | 3,3 | 0 | 4,1 |

Gegenüber den unbehandelten Stoffen und den mit einer Standard-Silicon-Emulsion behandelten Stoffen werden der Weichgriff und die Wasseraufnahme durch die erfindungsgemäß eingesetzten Siloxancopolymere erheblich verbessert.

Beispiel 13: Tissueanwendung

Die erfindungsgemäßen wässrigen Dispersionen der Herstellungsbeispiele 7 und 10, sowie deren Gemisch (75% Beispiel 7 + 25% Beispiel 10) wurden im Vergleich zu den Wacker Standardprodukten Finish CT 34 E (= Vergleich 4) und WETSOFT® CTA (= Vergleich 3) auf Weichgriff und Saugfähigkeit auf Tissuepapier getestet.

Hierzu wurde das Tissuepapier mit 1,7% des jeweiligen Siliconwirkstoffs (0,85% je Seite) gleichmäßig beschichtet. Der Auftrag erfolgte über ein Dreiwalzenbeschichtungsgerät. Dazu wird die zu prüfende Emulsion in einen Vorlagebehälter gefüllt und von einer Gravurwalze aufgenommen. Die Gravurwalze überträgt die Emulsion auf eine Auftragswalze, welche die Emulsion gleichmäßig auf das Papier aufbringt. Das Papier wird hierzu auf einem Träger zwischen Auftrags- und einer Andruckwalze eingeschoben. Der Vorgang wird für die Beschichtung der anderen Papierseite wiederholt.

Das zur Prüfung herangezogene Tissuepapier ist eine auf dem Markt erhältliche, sehr saugfähige und offenporige Tissuequalität, so dass die beschichteten Papiere in Bezug auf Saugfähigkeit fast keine Differenzierung aufwiesen.

Prüfung des Weichgriffs:

Eine Reihe von unterschiedlich beschichteten Tissuepapieren wird durch Anfassen untereinander und mit einem unbeschichteten Papier (Blindwert) verglichen.

Eine Person vergleicht durch Greifen mit den Fingern (Hautkontakt) 2 Tissuepapiere gleicher Größe miteinander und entscheidet, welches Tissuepapier sich weicher und angenehmer anfühlt, oder ob kein Unterschied besteht.
Das weichere Papier bekommt 1 Punkt, das schlechtere 0 Punkte, besteht kein greifbarer Unterschied, bekommen beide Papiere 0,5 Punkte.
Nach dieser Vorgehensweise werden alle beschichteten Tissuepapiere und der Blindwert untereinander verglichen.
Bei der Beurteilung gemäß Tabelle 3 wurden 5 mit unterschiedlichen Produkten beschichtete Papiere und der Blindwert von insgesamt 4 Testpersonen geprüft, d.h. das Produkt mit dem besten Weichgriff kann max. 20 Punkten, das mit dem schlechtesten 0 Punkte bekommen.

Tropfentest:

Um die Wasseraufnahme eines beschichteten Tissuepapiers zu bestimmen wird der sog. Tropfentest durchgeführt. Hierzu werden die zu testenden Papiere in einen Rahmen eingespannt, so dass sich keine Falten bilden (Blechdeckel mit Spannring), und aus einer Höhe von 1 cm wird ein Tropfen Wasser aus einer Bürette mit einer Tropfgeschwindigkeit von 5s/Tropfen auf das zu prüfende Tissuepapier aufgebracht.
Die Zeit bis zum völligen Verschwinden des Wassertropfens wird notiert.
Dieser Test wird insgesamt 4 mal (2 mal je Seite) an verschiedenen Stellen des Tissuepapiers durchgeführt. Angegeben wird dann der Mittelwert aus den 4 Messungen.

Die Ergebnisse sind in Tabelle 3 zusammengefasst.

Tabelle 3:

| Silicon-emulsion | Blind-wert (unbeh.) | CTA (Vergleich 3) | WR 1100 (Vergleich 4) | Beispiel 10 | 75% Beispiel 7 25% Beispiel 10 | Beispiel 7 |
|---|---|---|---|---|---|---|
| Weichgriff | 0 | 7,0 | 11,0 | 11,5 | 16,5 | 15,5 |
| Tropfentest [in s] | 1,1 | 1,7 | 1, 6 | 1, 4 | 1,2 | 1, 3 |

Die beiden Vergleichsprodukte WR 1100 und CTA waren in Bezug auf Weichgriff den erfindungsgemäßen Siloxancopolymeren gemäß den Beispielen 7 und 10 und deren Mischung deutlich unterlegen. Beim Tropfentest, d. h. bei der Wasseraufnahme und Saugfähigkeit eines Tissuepapiers, lagen alle Produkte in einer ähnlichen Größenordnung, aber auch hier zeigten die erfindungsgemäßen Siloxancopolymere eher eine bessere Wasseraufnahme als die Vergleichsbeispiele.

**Patentansprüche**

1. Verfahren zur Modifizierung faserartiger Substrate mit Siloxancopolymeren herstellbar indem in einem ersten Schritt
Organopolysiloxane (1), die pro Molekül mindestens ein Si-gebundenes Wasserstoffatom aufweisen, mit weitgehend linearen oligomeren oder polymeren Verbindungen (2) der allgemeinen Formel

$$R^1\text{-}(A\text{-}C_nH_{2n})_m\text{-}A^1\text{-}H \qquad (I),$$

wobei $R^1$ einen einwertigen gegebenenfalls substituierten Kohlenwasserstoffrest, an den Si-H-Gruppen in einer Hydrosilylierungsreaktion angelagert werden können, bedeutet,

A einen zweiwertigen, polaren organischen Rest ausgewählt aus der Gruppe von -O- , -C(O)-O- , -O-C(O)- , -O-C(O)-O-, -C(O)-NH- , -NH-C(O)- , Urethanrest und Harnstoffrest bedeutet,

$A^1$ einen zweiwertigen, polaren organischen Rest ausgewählt aus der Gruppe von -O- , -NH- und -NR'- (wobei R' einen einwertigen Kohlenwasserstoffrest mit 1 bis 18 Kohlenstoffatomen bedeutet) bedeutet,

n eine ganze Zahl von 1 bis 20 ist und

m gleich 0 oder eine ganze positive Zahl ist,

umgesetzt werden,

und in einem zweiten Schritt

die so erhaltenen $H-A^1$-Gruppen auf weisenden Zwischenprodukte (3) mit organischen Verbindungen (4), die pro Molekül mindestens zwei Isocyanatgruppen aufweisen, umgesetzt werden.

2. Verfahren nach Anspruch 1, **dadurch gekennzeichnet, dass** der Wassergehalt der zur Herstellung der Siloxancopolymere eingesetzten Verbindungen (1) und (2) niedriger ist als 2000 Gew.-ppm, jeweils bezogen auf das Gesamtgewicht von Verbindungen (1) und (2).

3. Verfahren nach Anspruch 1 oder 2, **dadurch gekennzeichnet, dass** die organischen Verbindungen (4), die pro Molekül mindestens zwei Isocyanatgruppen aufweisen, in Mengen von 0,5 bis 1,0 Mol Isocyanatgruppe je Mol $H-A^1$-Gruppe im Zwischenprodukt (3) eingesetzt werden.

4. Verfahren nach Anspruch 1, 2 oder 3, **dadurch gekennzeichnet, dass** als Organopolysiloxane (1) solche der allgemeinen Formel

$$H_gR_{3-g}SiO(SiR_2O)_o(SiRHO)_pSiR_{3-g}H_g \qquad (III)$$

wobei R gleich oder verschieden sein kann und einen einwertigen, gegebenenfalls substituierten Kohlenwasserstoffrest mit 1 bis 18 Kohlenstoffatomen je Rest bedeutet,

g 0, 1 oder 2,

o 0 oder eine ganze Zahl von 1 bis 1500 und

p 0 oder eine ganze Zahl von 1 bis 200 ist,

mit der Maßgabe, dass pro Molekül mindestens ein Si-gebundenes Wasserstoffatom, vorliegt,

eingesetzt werden.

5. Verfahren nach einem der Ansprüche 1 bis 4, **dadurch gekennzeichnet, dass** die Organopolysiloxane (1) $\alpha,\omega$-Dihydrogendiorganopolysiloxane sind.

6. Verfahren nach einem der Ansprüche 1 bis 5, **dadurch gekennzeichnet, dass** A und $A^1$ in Formel (I) ein Sauerstoffatom -O- sind.

7. Verfahren nach einem der Ansprüche 1 bis 6, **dadurch gekennzeichnet, dass** die Verbindung (2) ein aliphatisch ungesättigter Alkohol der allgemeinen Formel

$$H_2C=CH-R^2-(OC_nH_{2n})_m-OH \qquad (IV),$$

wobei $R^2$ ein zweiwertiger Kohlenwasserstoffrest mit 1 bis 10 Kohlenstoffatomen ist und

n und m die in Anspruch 1 angegebene Bedeutung haben, ist.

8. Verfahren nach einem der Ansprüche 1 bis 7, **dadurch gekennzeichnet, dass** die Verbindung (4) ein Diisocyanat der allgemeinen Formel

$$O=C=N-R^3-N=C=O \qquad (V),$$

wobei $R^3$ einen zweiwertigen Kohlenwasserstoffrest mit 4 bis 40 Kohlenstoffatomen je Rest bedeutet,

ist.

9. Verfahren nach einem der Ansprüche 1 bis 8, **dadurch gekennzeichnet, dass** im zweiten Verfahrensschritt als weitere Verbindung (7) solche ausgewählt aus der Gruppe der Formeln

$$R^4- (A-C_nH_{2n})_m-A^1-H \qquad (VII),$$

$$HO-R^5-NR^4-R^5-OH \qquad (VIII),$$

$$HO-R^5-NR^4_2 \qquad (IX),$$

$$HO-R^6(NR^4_2)_2 \qquad (X),$$

$$HO-R^7(NR^4_2)_3 \qquad (XI),$$

$$(HO)_2R^6-NR^4_2 \qquad (XII)$$

und

$$HNR^4_2 \qquad (XIII)$$

wobei $R^4$ ein Wasserstoffatom oder einen Rest R, der gegebenenfalls ein Stickstoffatom enthalten kann, bedeutet,
$R^5$ einen zweiwertigen Kohlenwasserstoffrest mit 1 bis 10 Kohlenstoffatomen je Rest bedeutet,
$R^6$ einen dreiwertigen organischen Rest mit 1 bis 100 Kohlenstoffatomen je Rest, vorzugsweise einen dreiwertigen Kohlenwasserstoffrest mit 1 bis 100 Kohlenstoffatomen, der ein oder mehrere Sauerstoffatome enthält,
$R^7$ einen vierwertigen organischen Rest mit 1 bis 100 Kohlenstoffatomen je Rest, vorzugsweise einen vierwertigen Kohlenwasserstoffrest mit 1 bis 100 Kohlenstoffatomen, der ein oder mehrere Sauerstoffatome enthält, und
$A^1$, n und m die im Anspruch 1 dafür angegebene Bedeutung haben,
mitverwendet werden.

10. Verfahren nach Anspruch 9, **dadurch gekennzeichnet, dass** die organischen Verbindungen (4) in Mengen von 0,5 bis 1,0 Mol Isocyanatgruppe je Mol der Summe mit Isocyanatgruppen reagierender Gruppen aus der Summe von Zwischenprodukt (3) und Verbindungen (7) eingesetzt werden.

11. Verfahren nach einem der Ansprüche 1 bis 8, **dadurch gekennzeichnet, dass** als Siloxancopolymere solche der allgemeinen Formel

$$CH_2=CH- R^3-(OC_nH_{2n})_m-OC (O) NH- R^2-NHC (O) O[(C_nH_{2n}O)_m-R^3-CH_2CH_2-R_2SiO (R_2SiO)_o-$$
$$R_2SiO-CH_2CH_2-R^3-(OC_nH_{2n})_m-OC(O)NH-R^2-NHC(O)O]_x(C_nH_{2n}O)_m-R^3-CH=CH_2 \qquad (VI),$$

wobei R gleich oder verschieden sein kann und einen einwertigen, gegebenenfalls substituierten Kohlenwasserstoffrest mit 1 bis 18 Kohlenstoffatomen je Rest bedeutet,
$R^2$ ein zweiwertiger Kohlenwasserstoffrest mit 1 bis 10 Kohlenstoffatomen,
$R^3$ einen zweiwertigen Kohlenwasserstoffrest mit 4 bis 40 Kohlenstoffatomen je Rest bedeutet,
n eine ganze Zahl von 1 bis 20 ist und
m gleich 0 oder eine ganze positive Zahl ist,
o 0 oder eine ganze Zahl von 1 bis 1500 und
x 0 oder eine ganze Zahl von 1 bis 20 ist,
eingesetzt werden.

12. Verfahren nach einem der Ansprüche 1 bis 11 **dadurch gekennzeichnet, dass** die Siloxancopolymere in Form von wässrigen Emulsionen oder Microemulsionen eingesetzt werden.

13. Verfahren nach einem der Ansprüche 1 bis 12, **dadurch gekennzeichnet, dass** textile Flächengebilde oder Fasern oder Garne modifiziert werden.

14. Verfahren nach einem der Ansprüche 1 bis 12, **dadurch gekennzeichnet, dass** die Siloxancopolymere als Weichspüler oder Weichmacher von textilen Flächengebilden oder Textilien eingesetzt werden.

15. Verfahren nach einem der Ansprüche 1 bis 12, **dadurch gekennzeichnet, dass** Papier modifiziert wird.

**16.** Verfahren nach einem der Ansprüche 1 bis 12 **dadurch gekennzeichnet, dass** Haare modifiziert oder behandelt werden.

**17.** Verfahren nach einem der Ansprüche 1 bis 12, **dadurch gekennzeichnet, dass** die Siloxancopolymere in Haarshampoo oder Haarpflegemittel oder Haarconditioner eingesetzt werden.

**18.** Verfahren nach einem der Ansprüche 1 bis 12, **dadurch gekennzeichnet, dass** Leder modifiziert wird.

**19.** Verfahren nach einem der Ansprüche 1 bis 12, **dadurch gekennzeichnet, dass** die Siloxancopolymere in Lacken oder Lasuren eingesetzt werden.

**20.** Faserartige Substrate, **dadurch gekennzeichnet, dass** sie nach dem Verfahren gemäß den Ansprüchen 1 bis 12 modifiziert wurden.

**21.** Faserartige Substrate nach Anspruch 20, **dadurch gekennzeichnet, dass** es sich um textile Flächengebilde, Fasern, Garne, Papier oder Leder handelt.

**Claims**

**1.** Method of modifying fibrous substrates with siloxane copolymers obtainable by
a first step of reacting
organopolysiloxanes (1) which have at least one silicon-attached hydrogen atom per molecule with substantially linear oligomeric polymeric compounds (2) of the general formula

$$R^1\text{-}(A\text{-}C_nH_{2n})_m\text{-}A^1\text{-}H \qquad (I)$$

where $R^1$ is a univalent optionally substituted hydrocarbyl radical capable of adding Si-H groups in a hydrosilylation reaction,
A is a bivalent polar organic radical selected from the group consisting of -O-, -C(O)-O-, -O-C(O)-, -O-C(O)-O-, -C(O)-NH-, -NH-C(O)-, urethane radical and urea radical,
$A^1$ is a bivalent polar organic radical selected from the group consisting of -O-, -NH- and -NR'-(where R' is a univalent hydrocarbyl radical of 1 to 18 carbon atoms),
n is an integer from 1 to 20, and
m is equal to 0 or a positive integer,
and a second step of reacting
the resulting $H\text{-}A^1$-containing intermediates (3) with organic compounds (4), which have two or more isocyanate groups per molecule.

**2.** Method according to Claim 1, **characterized in that** the water content of the compounds (1) and (2) used for preparing the siloxane copolymers is lower than 2000 weight ppm, in each case based on the total weight of compounds (1) and (2).

**3.** Method according to Claim 1 or 2, **characterized in that** the organic compounds (4), which have two or more isocyanate groups per molecule, are used in amounts of 0.5 to 1.0 mol of isocyanate group per mole of $H\text{-}A^1$ group in the intermediate (3).

**4.** Method according to Claim 1, 2 or 3, **characterized in that** said organopolysiloxanes (1) have the general formula

$$H_gR_{3\text{-}g}SiO\ (SiR_2O)_o\ (SiRHO)\ pSiR_{3\text{-}g}H_g \qquad (III)$$

where R in each occurrence may be the same or different and is a univalent optionally substituted hydrocarbyl radical having 1 to 18 carbon atoms per radical,
g is 0, 1 or 2,
o is 0 or an integer from 1 to 1500, and
p is 0 or an integer from 1 to 200,
with the proviso that there is at least one silicon-attached hydrogen atom per molecule.

5. Method according to any one of Claims 1 to 4, **characterized in that** said organopolysiloxanes (1) are $\alpha,\omega$-dihydrodiorganopolysiloxanes.

6. Method according to any one of Claims 1 to 5, **characterized in that** A and $A^1$ in the formula (I) are an oxygen atom -O-.

7. Method according to any one of Claims 1 to 6, **characterized in that** said compound (2) is an aliphatically unsaturated alcohol of the general formula

$$H_2C=CH-R^2-(OC_nH_{2n})_m-OH \qquad (IV)$$

where $R^2$ is a bivalent hydrocarbyl radical of 1 to 10 carbon atoms and
n and m are each as defined in Claim 1.

8. Method according to any one of Claims 1 to 7, **characterized in that** said compound (4) is a diisocyanate of the general formula

$$O=C=N-R^3-N=C=O \qquad (V)$$

where $R^3$ is a bivalent hydrocarbyl radical having 4 to 40 carbon atoms per radical.

9. Method according to any one of Claims 1 to 8, **characterized in that** the second step utilizes further compounds (7) selected from the group of the formulae

$$R^4-(A-C_nH_{2n})_m-A^1-H \qquad (VII),$$

$$HO-R^5-NR^4-R^5-OH \qquad (VIII),$$

$$HO-R^5-NR^4_2 \qquad (IX),$$

$$HO-R^6(NR^4_2)_2 \qquad (X),$$

$$HO-R^7(NR^4_2)_3 \qquad (XI),$$

$$(HO)_2R^6-NR^4_2 \qquad (XII)$$

and

$$HNR^4_2 \qquad (XIII)$$

where $R^4$ is a hydrogen atom or an R radical which may optionally contain a nitrogen atom,
$R^5$ is a bivalent hydrocarbyl radical having 1 to 100 carbon atoms per radical,
$R^6$ is a tervalent organic radical having 1 to 100 carbon atoms per radical, preferably a tervalent hydrocarbyl radical of 1 to 100 carbon atoms, which contains one or more oxygen atoms,
$R^7$ is a tetravalent organic radical having 1 to 100 carbon atoms per radical, preferably a tetravalent hydrocarbyl radical of 1 to 100 carbon atoms, which contains one or more oxygen atoms, and
$A^1$, n and m are each as defined in claim 1.

10. Method according to Claim 9, **characterized in that** said organic compounds (4) are used in amounts of 0.5 to 1.0 mol of isocyanate group per mole of the sum total of isocyanate-reactive groups from the sum total of intermediate (3) and compounds (7).

11. Method according to any one of Claims 1 to 8, **characterized in that** siloxane copolymers used have the general formula

$$CH_2=CH-R^3-(OC_nH_{2n})_m-OC(O)NH-R^2-NHC(O)O[(C_nH_{2n}O)_m-R^3-CH_2CH_2-R_2SiO(R_2SiO)_o-$$
$$R_2SiO-CH_2CH_2-R^3-(OC_nH_{2n})_m-OC(O)NH-R^2-NHC(O)O]_x(C_nH_{2n}O)_m-R^3-CH=CH_2 \qquad (IV)$$

where R in each occurrence may be the same or different and is a univalent, optionally substituted hydrocarbyl radical having 1 to 18 carbon atoms per radical,
$R^2$ is a bivalent hydrocarbyl radical of 1 to 10 carbon atoms,
$R^3$ is a bivalent hydrocarbyl radical having 4 to 40 carbon atoms per radical,
n is an integer from 1 to 20,
m is equal to 0 or a positive integer,
o is 0 or an integer from 1 to 1500, and
x is 0 or an integer from 1 to 20.

12. Method according to any one of Claims 1 to 11, **characterized in that** the siloxane copolymers are used in the form of aqueous emulsions or microemulsions.

13. Method according to any one of Claims 1 to 12, **characterized in that** textile sheet materials or fibers or yarns are modified.

14. Method according to any one of Claims 1 to 12, **characterized in that** the siloxane copolymers are used as softeners for textile sheet materials or textiles, including softeners applied in the final rinse cycle of a washing machine.

15. Method according to any one of Claims 1 to 12, **characterized in that** paper is modified.

16. Method according to any one of Claims 1 to 12, **characterized in that** hair is modified or treated.

17. Method according to any one of Claims 1 to 12, **characterized in that** the siloxane copolymers are used in hair shampoo or haircare product or hair conditioner.

18. Method according to any one of Claims 1 to 12, **characterized in that** leather is modified.

19. Method according to any one of Claims 1 to 12, **characterized in that** the siloxane copolymers are used in coatings or glazes.

20. Fibrous substrates, **characterized in that** they have been modified by the method according to Claims 1 to 12.

21. Fibrous substrates according to Claim 20, **characterized in that** they comprise textile sheet materials, fibers, yarns, paper or leather.

**Revendications**

1. Procédé de modification de substrats fibreux avec des copolymères de siloxane pouvant être obtenus en ce que :

dans une première étape,
des organopolysiloxanes (1), qui renferment au moins un atome d'hydrogène lié au silicium par molécule, sont mis à réagir avec
des composés oligomères ou polymères essentiellement linéaires (2) de formule générale :

$$R^1\text{-}(A\text{-}C_nH_{2n})_m\text{-}A^1\text{-}H \qquad (I)$$

dans laquelle $R^1$ représente un radical hydrocarboné monovalent, éventuellement substitué, sur lequel des groupes Si-H peuvent être additionnés dans une réaction d'hydrosilylation,
A représente un radical organique polaire bivalent choisi parmi le groupe constitué d'un groupe -O-, d'un groupe -C(O)-O-, d'un groupe -O-C(O)-, d'un groupe -O-C(O)-O-, d'un groupe -C(O)-NH-, d'un groupe -NH-C(O)-, d'un radical uréthane et d'un radical urée,
$A^1$ représente un radical organique polaire bivalent choisi parmi le groupe constitué d'un groupe -O-, d'un groupe -NH- et d'un groupe -NR'- (dans lequel R' représente un radical hydrocarboné monovalent renfermant de 1 à 18 atomes de carbone),
n est un entier de 1 à 20, et
m vaut 0 ou est un entier positif,
et, dans une deuxième étape,
les intermédiaires renfermant des groupes H-$A^1$ (3) ainsi obtenus sont mis à réagir avec des composés organiques (4) qui renferment au moins deux groupes isocyanate par molécule.

2. Procédé selon la revendication 1, **caractérisé en ce que** la teneur en eau des composés (1) et (2) utilisés pour la préparation des copolymères de siloxane est inférieure à 2 000 ppm en poids, dans chaque cas par rapport au poids total des composés (1) et (2).

3. Procédé selon la revendication 1 ou 2, **caractérisé en ce que** les composés organiques (4), qui renferment au moins deux groupes isocyanate par molécule, sont utilisés en des quantités de 0,5 à 1,0 mol de groupe isocyanate par mole de groupe H-A$^1$ dans l'intermédiaire (3).

4. Procédé selon la revendication 1, 2 ou 3, **caractérisé en ce que** l'on utilise, en tant qu'organopolysiloxanes (1), ceux de formule générale :

$$H_gR_{3-g}SiO\ (SiR_2O)_o(SiRHO)_pSiR_{3-g}H_g \qquad (III)$$

dans laquelle R peut être identique ou différent et représente un radical hydrocarboné monovalent, éventuellement substitué, renfermant de 1 à 18 atomes de carbone par radical,
g vaut 0, 1 ou 2,
o vaut 0 ou est un entier de 1 à 1 500, et
p vaut 0 ou est un entier de 1 à 200,
à condition qu'au moins un atome d'hydrogène lié au silicium soit présent par molécule.

5. Procédé selon l'une quelconque des revendications 1 à 4, **caractérisé en ce que** les organopolysiloxanes (1) sont des $\alpha,\omega$-dihydrogénodiorganopolysiloxanes.

6. Procédé selon l'une quelconque des revendications 1 à 5, **caractérisé en ce que** A et A$^1$ dans la formule (I) sont un atome d'oxygène -O-.

7. Procédé selon l'une quelconque des revendications 1 à 6, **caractérisé en ce que** le composé (2) est un alcool aliphatiquement insaturé de formule générale :

$$H_2C{=}CH{-}R^2{-}(OC_nH_{2n})_m{-}OH \qquad (IV)$$

dans laquelle R$^2$ représente un radical hydrocarboné bivalent renfermant de 1 à 10 atomes de carbone, et n et m présentent la signification indiquée dans la revendication 1.

8. Procédé selon l'une quelconque des revendications 1 à 7, **caractérisé en ce que** le composé (4) est un diisocyanate de formule générale :

$$O{=}C{=}N{-}R^3{-}N{=}C{=}O \qquad (V)$$

dans laquelle R$^3$ représente un radical hydrocarboné bivalent renfermant de 4 à 40 atomes de carbone par radical.

9. Procédé selon l'une quelconque des revendications 1 à 8, **caractérisé en ce que**, dans la deuxième étape du procédé, on utilise conjointement, en tant que composé supplémentaire (7), ceux choisis parmi le groupe de formules :

$$R^4{-}(A{-}C_nH_{2n})_m{-}A^1{-}H \qquad (VII),$$

$$HO{-}R^5{-}NR^4{-}R^5{-}OH \qquad (VIII),$$

$$HO{-}R^5{-}NR^4_2 \qquad (IX),$$

$$HO{-}R^6(NR^4_2)_2 \qquad (X),$$

$$HO{-}R^7(NR^4_2)_3 \qquad (XI),$$

$$(HO)_2R^6{-}NR^4_2 \qquad (XII)$$

et

$$HNR^4_2 \qquad (XIII)$$

dans lequelles $R^4$ représente un atome d'hydrogène ou un radical R qui peut éventuellement renfermer un atome d'azote,

$R^5$ représente un radical hydrocarboné bivalent renfermant de 1 à 10 atomes de carbone par radical,

$R^6$ représente un radical organique trivalent renfermant de 1 à 100 atomes de carbone par radical, de préférence un radical hydrocarboné trivalent renfermant de 1 à 100 atomes de carbone, qui renferme un ou plusieurs atomes d'oxygène,

$R^7$ représente un radical organique tétravalent renfermant de 1 à 100 atomes de carbone par radical, de préférence un radical hydrocarboné tétravalent renfermant de 1 à 100 atomes de carbone, qui renferme un ou plusieurs atomes d'oxygène, et

$A^1$, n et m présentent la signification indiquée à cet effet dans la revendication 1.

10. Procédé selon la revendication 9, **caractérisé en ce que** les composés organiques (4) sont utilisés en des quantités de 0,5 à 1,0 mol de groupe isocyanate par mole de la somme des groupes réagissant avec des groupes isocyanate provenant de la somme de l'intermédiaire (3) et des composés (7).

11. Procédé selon l'une quelconque des revendications 1 à 8, **caractérisé en ce que** l'on utilise, en tant que copolymères de siloxane, ceux de formule générale :

$$CH_2=CH-R^3-(OC_nH_{2n})_m-OC(O)NH-R^2-NHC(O)O[(C_nH_{2n}O)_m-R^3-CH_2CH_2R_2SiO(R_2SiO)_o-$$
$$R_2SiO-CH_2CH_2-R^3-(OC_nH_{2n})_m-OC(O)NH-R^2-NHC(O)O]_x(C_nH_{2n}O)_m-R^3-CH=CH_2 \qquad (IV)$$

dans laquelle R peut être identique ou différent et représente un radical hydrocarboné monovalent, éventuellement substitué, renfermant de 1 à 18 atomes de carbone par radical,

$R^2$ représente un radical hydrocarboné bivalent renfermant de 1 à 10 atomes de carbone,

$R^3$ représente un radical hydrocarboné bivalent renfermant de 4 à 40 atomes de carbone par radical,

n est un entier de 1 à 20, et

m vaut 0 ou est un entier positif,

o vaut 0 ou est un entier de 1 à 1 500, et

x vaut 0 ou est un entier de 1 à 20.

12. Procédé selon l'une quelconque des revendications 1 à 11, **caractérisé en ce que** les copolymères de siloxane sont utilisés sous la forme d'émulsions ou de microémulsions aqueuses.

13. Procédé selon l'une quelconque des revendications 1 à 12, **caractérisé en ce que** des structures plates textiles ou des fibres ou des fils sont modifiés.

14. Procédé selon l'une quelconque des revendications 1 à 12, **caractérisé en ce que** les copolymères de siloxane sont utilisés en tant qu'assouplisseurs ou adoucissants de structures plates textiles ou de textiles.

15. Procédé selon l'une quelconque des revendications 1 à 12, **caractérisé en ce que** du papier est modifié.

16. Procédé selon l'une quelconque des revendications 1 à 12, **caractérisé en ce que** des cheveux sont modifiés ou traités.

17. Procédé selon l'une quelconque des revendications 1 à 12, **caractérisé en ce que** les copolymères de siloxane sont utilisés dans des shampooings pour les cheveux ou des produits de soins des cheveux ou des conditionneurs de cheveux.

18. Procédé selon l'une quelconque des revendications 1 à 12, **caractérisé en ce que** du cuir est modifié.

19. Procédé selon l'une quelconque des revendications 1 à 12, **caractérisé en ce que** les copolymères de siloxane sont utilisés dans des revêtements ou des glacis.

**20.** Substrats fibreux **caractérisés en ce qu'**ils ont été modifiés conformément au procédé selon les revendications 1 à 12.

**21.** Substrats fibreux selon la revendication 20, **caractérisés en ce qu'**il s'agit de structures plates textiles, de fibres, de fils, de papier ou de cuir.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5001210 A
- EP 1178069 A
- US 20030032726 A
- WO 02088209 A, A. Andrew Shores
- US 20030032751 A, A. Andrew Shores
- US 3775452 A

**In der Beschreibung aufgeführte Nicht-Patentliteratur**

- *CHEMICAL ABSTRACTS,* 136: 38808